# EUROPEAN PATENT APPLICATION

(11) **EP 1 925 322 A1**
(43) Date of publication of application: **28.05.2008**
(21) Application number: 06077105.2
(22) Date of filing: 27.11.2006
(51) Int. Cl.: A61L 9/12

(54) **Fragrance generator**

(71) Applicant: Sara Lee/DE N.V., 3532 AA Utrecht (NL)
(72) Inventor: Miro Amenos, Jordi, 08027 Barcelona (ES); Feliu Fontaba, Carlos, 08004 Barcelona (ES); Vilana Balastegui, Joan, 08191-Rubi, Barcelona (ES)
(74) Representative: van Loon, C.J.J.

(57) **Abstract**

A fragrance generator comprises a housing for containing a volatile liquid to provide a vapour. The housing has a wall of which at least a part is permeable to the vapour. The generator further comprises an actuator for diffusing the vapour emerging from the volatile liquid to an environment outside the housing. The generator is provided with an electric circuit comprising a solar energy converter arranged to convert solar energy into electric energy for feeding the actuator and the circuit comprises a capacitor for accumulating the electric energy from the solar energy converter.

## Description

The invention relates to a fragrance generator.

A fragrance generator is, for instance, known from British patent application GB - A - 2 247 623. The fragrance generator may be provided for dispensing, in vapour form, a perfume, a fragrance or another substance. A housing is provided having an aperture permitting the egress of air and vapour from the interior of the housing. The apparatus further comprises a fan positioned in the housing in relation to the inlet so that, when the motor is turned on, it creates airflow across the holder of the volatile liquid and discharges air through the aperture. The apparatus also comprises a rechargeable battery and a solar cell connected thereto via an electrical charging circuit.

It is significantly less likely that a person is present in a room during night time, than during daytime. Even so it is less likely that a person is present in a room when the lights are switched off than when the lights are switched on. This particularly applies to living rooms. Therefore, a disadvantage of the known apparatus is the fact that the motor will continue to create the air flow even during the night time, even though nobody is likely to notice any of the vapour, which is dispensed by the apparatus.

Besides that, it is known that when a fragrance is released continuously a person present in the room will, at the end, not perceive the fragrance anymore. Varying the amount of fragrance released over time results in that the person in the room will perceive the fragrance over and over. A discontinuous release of fragrance is therefore advantageous.

Similarly, if the apparatus is used in a toilet, the apparatus will continue to create the air flow long after a person using the toilet has left the toilet and thus the apparatus will continue to dispense the vapour much longer than required.

It is desired to provide the possibility to obviate one or more of the above disadvantages.

According to an aspect of the invention, a fragrance generator is provided, the fragrance generator comprising a housing for containing a volatile liquid to provide a vapour, the housing having a wall of which at least a part is permeable to the vapor, the fragrance generator further comprising an actuator for diffusing the vapour emerging from the volatile liquid to an environment outside the housing through the permeable wall part; wherein the fragrance generator is provided with an electric circuit comprising a solar energy converter arranged to convert solar energy into electric energy for feeding the actuator, wherein the circuit comprises a capacitor for accumulating the electric energy from the solar energy converter, the capacitor being associated with the actuator to provide the actuator with the electric energy.

The fragrance generator will now be further elucidated with reference to the drawings, wherein like reference numerals refer to like parts and wherein
Figure 1 is a schematic representation of a first embodiment of the fragrance generator according to the invention;
Figure 2 is a schematic representation of a second embodiment of the fragrance generator according to the invention;
Figure 3 is a schematic representation of a third embodiment of the fragrance generator according to the invention;
Figure 4 is a perspective view of a fourth embodiment of the fragrance generator according to the invention;
Figure 5 is an exploded view of a functional part of the fragrance generator shown in Figure 4;
Figure 6 is a perspective view of a body of the fragrance generator of Figure 4;
Figure 7 is a perspective view of a foot of the fragrance generator of Figure 4; and
Figure 8 is a perspective view of a refill component of the fragrance generator of Figure 4.

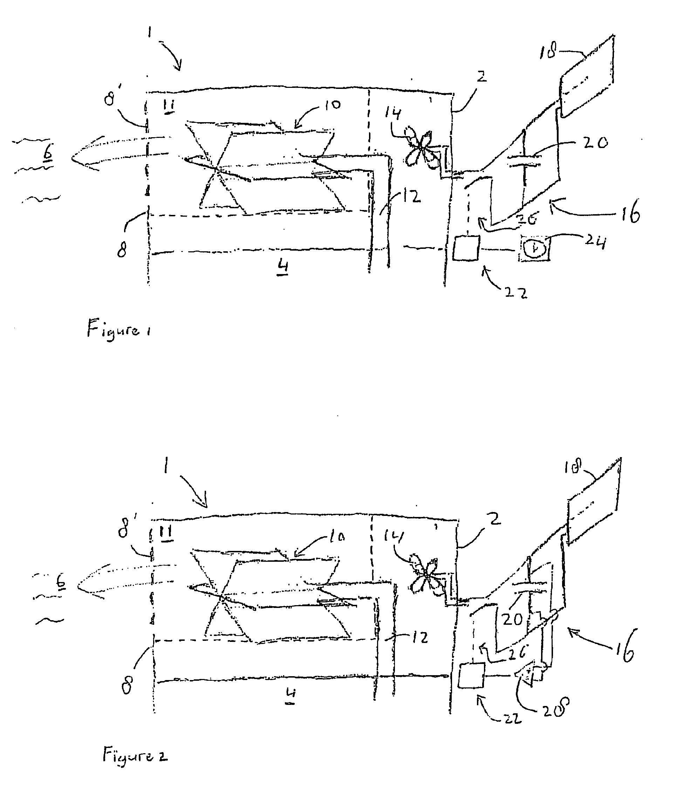

In Figure 1 a fragrance generator 1 is shown. The fragrance generator 1 may comprise a housing 2 for containing a volatile liquid 4 to provide a vapour 6 having a certain fragrance. The housing 2 has a wall 8 of which a part 8' is permeable to vapor, for instance, through a partly perforated wall as shown in the embodiment. Alternatively all of the wall 8 may be perforated.

The generator 1 may also comprise an evaporation surface 10 for enhancing an evaporation rate. The evaporation surface may be fixedly attached to the housing 2. Alternatively, a sub-compartment 11 may be provided in which the evaporation surface 10 is placed free from the housing as shown in the Figures. In order to transfer the volatile liquid 4 to the evaporation surface 10 the generator may be provided with a wick 12.

The generator 1 may further comprise an actuator 14, in this embodiment a fan 14, for diffusing the vapour emerging from the volatile liquid 4 to an environment outside the housing 2. The actuator 14 is, in this embodiment, arranged to diffuse the vapour emerging from the evaporation surface 10. In order to achieve this the actuator 14 may, as shown in Figure 1, be positioned to blow air in a direction parallel to an elongated direction of the evaporation surface 10.

Moreover, the generator 1 may be provided with an electric circuit 16 comprising a solar energy converter 18 arranged to convert solar energy into electric energy for feeding the actuator 14. The circuit 16 comprises a capacitor 20 for accumulating the electric energy from the solar energy converter 18, the capacitor 20 being associated with the actuator 14 to provide the actuator 14 with the electric energy.

The circuit 16 may comprise an activator 22 for intermittently allowing the capacitor 20 to provide the actuator 14 with the electric energy. The activator 22 is, in this embodiment, configured to provide the capacitor 16 with the electric energy at certain moments. For instance, the activator 22 may comprise a clock 24 and a relay 26. The activator 22 may further be configured to switch the relay into a closed position at set moments, in which closed position the capacitor 20 is allowed to provide the actuator 14 with the electric energy.

The fragrance generator 1 may be used in an enclosed space, such as a room, for instance in a toilet or in a living room. If a person enters the toilet, a light is usually turned on. As a consequence, the solar energy converter 18 commences to convert solar energy into electric energy which is stored in the capacitor 20. At a certain moment, the relay 26 is switched from an open to a closed position and the capacitor is allowed to provide the actuator 14 with the electric energy. The actuator 14 starts to rotate as a consequence thereby creating an air flow.

In the housing 2, the wick 12 sucks the volatile liquid 4 to the evaporation surface 10. The air flow transfers the vapour emerging from the evaporation surface 10 to the environment outside the housing 2 through the perforated part 8' of the wall 8, thereby spreading the fragrance.

After a certain time interval has elapsed, the relay 26 will switch back to the open position. Thus, the capacitor will be blocked from providing the actuator 14 with the electric energy. The fragrance is generally spread more effectively, if it is not performed continuously in time, but rather during certain intervals.

The capacitor 20 will, in this embodiment, not be recharged with electric energy when no one has been present in the toilet for some time, since the solar converter will not be provided with any light and the capacitor will be discharged soon after the solar converter has discontinued to convert solar energy into electric energy.

By the same token, the fragrance generator, if used in a living room, may, by applying the capacitor 20, be arranged refrain from dispensing the fragrance when the sun has set and/or the lights have been turned off, both of which may be strong indications that no one requiring the fragrance is present in the living room

A second embodiment of the fragrance generator 1 is shown in Figure 2. The second embodiment is quite similar to the first embodiment. However, in the second embodiment the activator 22 comprises a comparator 28. As the solar energy converter 18 provides the capacitor 20 with electric energy, the voltage on the capacitor 20 increases until it has reached a certain value. When the voltage exceeds the value, the relay 26 is switched to the closed position and the capacitor 20 is allowed to provide the actuator 14 with the electric energy, ultimately causing the vapour to emerge from the housing 2 and spreading the fragrance throughout the toilet.

When the voltage has dropped well below said value, the comparator 28 will cause the relay 26 to switch back to the open position. Thus, the capacitor will be blocked from providing the actuator 14 with the electric energy.

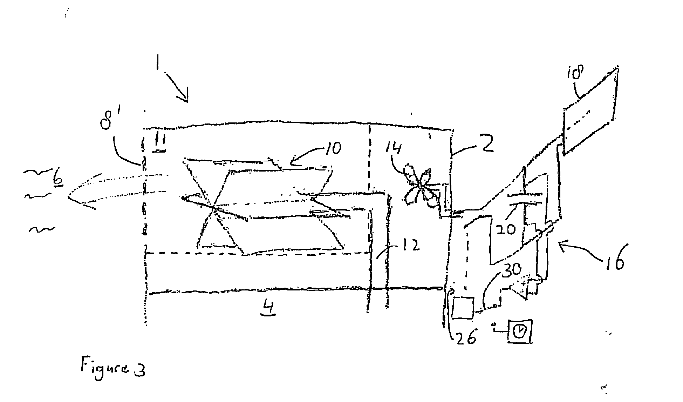

A third embodiment of the fragrance generator 1 is shown in Figure 3. The third embodiment may be seen as a combination of the first embodiment and the second embodiment and operates in a similar manner. The fragrance generator 1 comprises a switch 30 which may be in a first position, as shown in Figure 3, or in a second position. In the first position, the comparator 28 can switch the relay 26 and in the second position, the clock 24 can switch the relay 26.

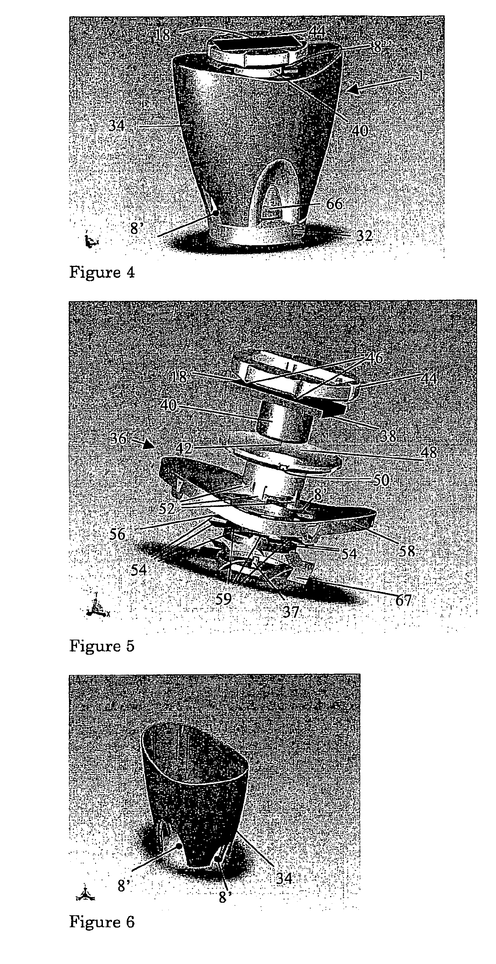

A fourth embodiment of the fragrance generator 1 is shown in Figures 4-8. The fragrance generator 1 comprises a foot 32 and a body 34 optionally made of plastics (see Figures 4, 5 and 6). The body 34 may comprise pins (not shown in the Figures) to hold the foot 32 and the foot 32 may comprise pin guides (not shown in the Figures) to guide the pins of the body 34.

The fragrance generator 1 further comprises a functional part 36, as can be seen in Figure 5. As shown in Figure 5, a fan blade 67 and solar energy converter 18 are comprised in the functional part 36. The fan blade 67 may comprise a hub 37, which forms an electrical circuit analogously to the electrical circuit 16 as disclosed in any one of the embodiments of Figure 1, 2 or 3. Furthermore, the functional part 36 comprises a printed circuit board 38 and on which the solar energy converter 18 may be located. A motor 40 for driving the fan blade 67 using a shaft 42 which may be structured to cooperate with the hub 37, wherein the motor 40 may be fixed to the printed circuit board 38, for instance, by way of soldering. In the depicted embodiment, fan blade 67, hub 37, shaft 42 and motor 40 form an actuator analogously to the actuator depicted in fig 1-3. The functional part 36 may, as in the embodiment of Figures 4-8, comprise an upper cover 44 having upper cover projections 46 and a lower cover 48 having lower cover notches 50 and lower cover clips 52. The lower cover notches 50 may fit into the upper cover projections 46, the upper cover 44 and the lower cover 48 together a holding the motor 40 and the printed circuit board 38.

Furthermore, the functional part 36 comprises a regulator 56 and a top piece 58 which, in the embodiment of Figures 4-8, is arranged to be fixed to the body 34. In the top piece 58, the cover 48 may be rotatably mounted and the top piece 58 comprises a perforated part 8' shown in both Figure 4 and Figure 5 provided as an air inlet. The regulator 56 may be fixed with respect to the lower cover 48. This may, for instance, be realised by having the lower cover clips 52 gripping regulator edges 59. The regulator 56 is located near said perforated part 8", and comprises lips 54 which are arranged on the regulator 56 such that the regulator 56 may be rotated with respect to the perforated part 8" such that the regulator 56 predetermines an effective area of the perforated part 8" through which an air flow caused by the actuator 14 is allowed. To rotate the regulator 56 with respect to the perforated part 8", a user may operate the upper cover 44 and the regulator 56 will be rotated along with the upper cover 44. The airflows through air inlet 8" and passes evaporation surface 10 before exiting through holes 8'.

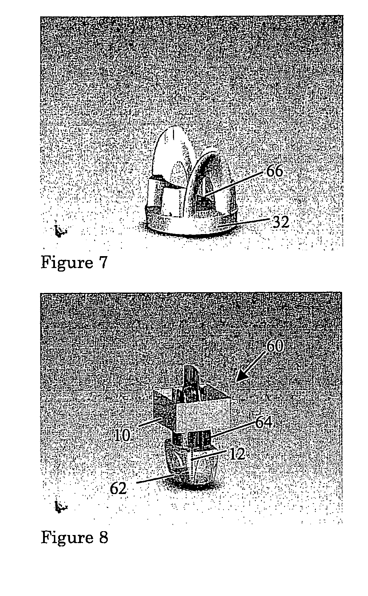

The fragrance generator of Figures 4-8 may further comprise, referring to Fig 8, a refillable and removable bottle 60 comprising a volatile liquid holder 62 serving as a sub-compartment for containing the volatile liquid 4 and a plug 64 having the wick 12 extending there through. The foot 32 may be provided with holes 66 to allow a user to see the bottle 60 and the level of volatile liquid 4 inside the bottle 60. In the embodiment of Figures 4-8, the volatile liquid holder 62 is a glass holder. However, any other suitable material may be used. The wick 12 extends from the volatile liquid holder 62 through the plug 64 to the evaporation surface 10 to transfer the volatile liquid 4 to the evaporation surface 10. The evaporation surface may suitably be oversized in order to provide for continuous evaporation. A suitable evaporation surface is one hundred square centimetre. Also, in order to concentrate the vapour emerging from the evaporation surface 10, the evaporation surface 10 may be enclosed inside the foot 32, the body 34 and the top piece 58.

To use the embodiment of the fragrance generator 1 shown in Figures 4-8, a cap may be removed from the bottle 60 if necessary, after which the plug 64 and the evaporation surface 10 may be mounted on the bottle 60. The bottle 60 is placed on the foot 32. Then the body 34 and the functional part 36 are placed on the foot 32. Using the wick 12, the evaporation surface 10 will be provided with the volatile liquid 4.

When there is enough light in a room in which the fragrance generator of Figures 4-8 is placed, the solar energy converter 18 captures the light and accumulates the power in the capacitor which is located on the printed circuit board 38 comprising a capacitor. The capacitor may, for example, activate the actuator 14 for a few seconds every few minutes, for instance five to ten seconds every five to ten minutes. In the embodiment shown in figs 4-8, preferably the capacitor is integrated on the printed circuit board 38 and functions in a similar way as described with reference to the fig 1-3 embodiments. In particular, preferably no other electric energy source is provided and the capacitor is used to directly provide the motor 40 with electrical energy when switched on. In addition, circuitry may be provided on the printed circuit board 38 to provide a clock similar to the clock 24 (depicted in fig 3 1-3). In addition, circuitry may be provided on the printed circuit board 38 to provide a relay similar to relay switch 22 (depicted in fig 3 1-3) to switch the capacitor on and off for providing electrical energy to motor 40. Moreover, in addition, circuitry may be provided on the printed circuit board 38 to provide a comparator similar to the comparator 28 depicted in fig 5 1-3 causing the relay switch to switch the capacitor on or off to motor 44 as a function of charged electrical energy. The actuator 14 may operate for ten seconds, then stop for ten minutes, then again operate for ten seconds etc. A similar mode operation may also used in the embodiment of Figure 1 or the embodiment of Figure 3.

It will be appreciated by a person skilled in the art that the invention is not limited to the embodiments disclosed here above. It is, for example, not strictly necessary to use the wick for transferring the volatile liquid to the evaporation surface. A transfer structure known to the skilled person. Also, it is not necessary to use a fan as an actuator for diffusing the vapour.

## Claims

1. Fragrance generator comprising a housing for containing a volatile liquid to provide a vapour, the housing having a wall of which at least a part is permeable to the vapour, the generator further comprising an actuator for diffusing the vapour to an environment outside the housing through at least the permeable wall part; wherein the fragrance generator is provided with an electric circuit comprising a solar energy converter arranged to convert solar energy into electric energy for feeding the actuator, wherein the circuit comprises a capacitor for accumulating the electric energy from the solar energy converter, the capacitor being associated with the actuator to provide the actuator with the electric energy.

2. Fragrance generator according to claim 1, wherein the circuit comprises an activator for intermittently allowing the capacitor to provide the actuator with the electric energy.

3. Fragrance generator according to claim 2, wherein the activator is configured to provide the actuator with the electric energy when a voltage over the capacitor exceeds a certain value.

4. Fragrance generator according to claim 2 or 3, wherein the activator is configured to provide the actuator with the electric energy at certain time intervals.

5. Fragrance generator according to any one of the preceding claims, further comprising an evaporation surface for enhancing an evaporation rate, the actuator being arranged to diffuse the vapour emerging from the evaporation surface.

6. Fragrance generator according to claim 5, further comprising a transfer structure for transferring the volatile liquid to the evaporation surface.

7. Fragrance generator according to claim 6, wherein the transfer structure is a wick.

8. Fragrance generator according to any one of the preceding claims, wherein the actuator is a fan.

9. Fragrance generator according to any one of the preceding claims, wherein the housing comprises a sub-compartment for containing the volatile liquid.

10. Fragrance generator according to claims 6 and 9, wherein the sub-compartment is provided with a plug and wherein the transfer structure extends through the plug.

11. Fragrance generator according to claim 9 or 10, wherein the sub-compartment of the housing is replaceable and/or refillable.

12. Fragrance generator according to any one of the preceding claims, wherein the fragrance generator comprises a regulator for regulating an effective area of the perforated part through which the vapour emerging from the volatile liquid diffuses to an environment outside the housing.
